# EUROPÄISCHE PATENTANMELDUNG

(11) **EP 0 898 961 A1**
(43) Veröffentlichungstag der Anmeldung: **03.03.1999**
(21) Anmeldenummer: 98116037.7
(22) Anmeldetag: 25.08.1998
(51) Int. Cl.: A61K 9/48, A61K 9/50, A61K 31/485, A61K 9/20

(54) **Pharmazeutische Zubereitung zur verbesserten Schmerztherapie bei akutem, postoperativem oder chronischem Schmerz**

(30) Priorität: 27.08.1997 DE 19737423
(71) Anmelder: Sahlender, Herta-Maria, Dr., 68723 Oftersheim (DE)
(72) Erfinder: Sahlender, Herta-Maria, Dr., 68723 Oftersheim (DE)
(74) Vertreter: Schwabe - Sandmair - Marx

(57) **Zusammenfassung**

Pharmazeutische Zubereitung zur Verwendung bei der Schmerztherapie von akutem, postoperativem oder chronischem Schmerz, die ein zur Schmerztherapie wirksames Pharmakon in retardierter und rasch freisetzender Form vorzugsweise in einem Verhältnis von 20 : 80 bis 80 : 20 und das Wirkungsverhältnis, bezogen auf die Schmerzintensität, von rasch freisetzender zu retardierender Form bei > 1 : 1 bis 20 : 1 liegt. Vorzugsweise handelt es sich bei dem Pharmakon um ein Analgetikum, ein Antiphlogistikum, ein Antirheumatikum oder einen nichtsteroidalen Entzündungshemmer, insbesondere um Morphin oder Codein.

## Beschreibung

Die vorliegende Erfindung betrifft das Gebiet der Schmerztherapie bei akutem, post-operativem oder chronischem Schmerz, insbesondere der Schmerztherapie bei Tumorpatienten.

Die Schmerztherapie chronischer Erkrankungen oder von andauernden Schmerzzuständen erfordert die Dauermedikation des Patienten. Um eine solche Dauermedikation zu erzielen, wird ein zur Schmerztherapie wirksames Pharmakon entweder kontinuierlich über einen Tropf oder eine Medikamentenpumpe verabreicht oder in Form retardierten Boluseinheiten appliziert.

Dauerhafte akute oder chronische Schmerzzustände sind ein eigenes Krankheitsbild und werden oft von Grunderkrankungen, wie z.B. der (rheumatischen) Polyarthritis, entzündlichen Erkrankungen, rheumatischen Erkrankungen aber auch von Tumoren verursacht. Zur Therapie der Schmerzzustände werden bislang, in Abhängigkeit von der Grunderkrankung und der Stärke des Schmerzes, übliche Analgetika, Antiphlogistika, Antirheumatika, nichtsteroidale Entzündungshemmer, Opioide und Opiate wie Ibuprofen, Metamizol, ASS, Paracetamol, Tramadol, Tilidin, Methadon, Buprenophin, Diclophenac und dessen Derivate, Codein und dessen Derivate sowie Morphin und dessen Derivate verabreicht.

Insbesondere bei Tumorpatienten ging man bisher von überwiegend gleicher Schmerzintensität im Tagesverlauf aus und strebte daher auch möglichst lineare Verläufe der Plasmakonzentration des Pharmakons an. Dazu werden dem Patienten retardierte Arzneimittelformen verabreicht, die den Wirkstoff verzögert über einen längeren Zeitraum freisetzen. Derartige Retardformulierungen sind z.B. aus den deutschen Offenlegungsschriften DE 41 22 039, 35 06 895, 22 24 534, 38 11 114 und 39 18 801 bekannt. Dabei kann, wie beispielsweise in der DE 39 18 801 beschrieben, vorgesehen sein, die retardierte Freisetzung durch eine schnell freisetzende Komponente zu ergänzen, die den im Tagesverlauf abgefallenen Plasmaspiegel des Pharmakons schnell wieder auf den gleichbleibend hohen Sollwert bringen soll.

Zur Schmerztherapie bei Tumorerkrankungen im Endstadium ist in den meisten Fällen die Verabreichung von Morphinen erforderlich. Allerdings birgt die erfolgreiche Schmerztherapie mit Opiaten, insbesondere den Morphinen, das Risiko von Nebenwirkungen wie Übelkeit, Schluckbeschwerden, Verdauungsbeschwerden (spastische Obstipation), Lähmung des Atemzentrums und nicht zuletzt die Suchtgefahr in sich.

Morphin und seine Derivate werden schnell (innerhalb von 10 bis 30 Minuten) resorbiert und weisen kurze Plasmahalbwertszeiten von nur wenigen Stunden auf. Die Schmerztherapie bei akutem, postoperativem und chronischem Schmerz erfolgt daher mit Retardformulierungen, meist Retardtabletten. Die derzeit übliche Art der Schmerzbehandlung mit diesen Retardtabletten richtet sich dabei mit der Plasmakonzentration des Pharmakons nach der Schmerzintensität. Bisher ging man von überwiegend gleicher Schmerzintensität im Tagesverlauf aus, weswegen möglichst lineare Verläufe der Plasmakonzentration an Morphin angestrebt wurden, gegebenenfalls mit Hilfe der oben genannten Ergänzung durch schnell freisetzende Komponenten zur schnellen Wiedereinstellung des konstant hohen Plasmaspiegels. Nachteil der dazu erforderlichen hohen Morphindosen sind die in erheblichem Umfang auftretenden Nebenwirkungen.

Es ist nun Aufgabe der vorliegenden Erfindung die Nebenwirkung von verschiedenen Therapeutika, insbesondere von Morphinen und Codeinen in der Schmerztherapie von akutem, postoperativem oder chronischem Schmerz zu verringern.

Der Lösung dieser Aufgabe liegt die Erkenntnis zu Grunde, daß die Schmerzintensitätskurve über den Tag hinweg nicht konstant ist, sondern morgens und abends Spitzenwerte (Peaks) aufweist. An diesen orientierte sich bislang die Therapie in den verabreichten Dosen. Erfindungsgemäß wird nun das Pharmakon in der Dosierung an den tatsächlichen Verlauf der Schmerzintensitätskurve angepaßt verabreicht. Technisch wird dies durch eine pharmazeutische Zubereitung gelöst, die das zur Schmerztherapie wirksame Pharmakon sowohl in retardierter Form als auch in rasch freisetzender Form enthält. Das Verhältnis von retardierter zu rasch freisetzender Form kann zwischen 20 : 80 bis 80 : 20 liegen. Vorzugsweise liegt das Verhältnis von 40 : 60 bis 60 : 40 und noch stärker bevorzugt bei 50 : 50. Das Wirkungsverhältnis bezogen auf die Schmerzintensität von rasch freisetzender zu retardierter Form liegt bei > 1 : 1 bis 20 : 1, vorzugsweise 2,5 : 1 bis 10 : 1 und noch stärker bevorzugt bei 3 : 1 bis 5 : 1. Die Galenik wird je nach Therapeutikum so gewählt, daß der durch die retardierte Form aufrechterhaltene Plasmaspiegel 20 bis 80 %, vorzugsweise 40 bis 75 % und am meisten bevorzugt 55 bis 65 % des auf 100 % gesetzten, durch die schnell freisetzende Form erzielten Plasmaspiegels beträgt.

Bei dem erfindungsgemäß verwendeten, zur Schmerztherapie wirksamen Pharmakon kann es sich um ein Analgetikum, ein Antirheumatikum, einen nichtsteroidalen Entzündungshemmer, Opioide und Opiate handeln. Als nicht beschränkende Beispiele seien genannt Acetylsalicylsäure, Ibuprofen, Paracetamol, Tramadol, Tilidin, Methadon, Buprenophin oder Metamizol, Diclophenac und dessen Derivate bzw. Codein und dessen Derivate oder Morphin und dessen Derivate und deren Gemische. Vorzugsweise handelt es sich um ein Morphin, ein Codein oder deren Derivate, noch stärker bevorzugt um Morphin und dessen Derivate. Die Verabreichung kann oral, parenteral (i.v., i.m. oder subcutan) oder transdermal bspw. über Pflaster erfolgen. Bei parenteralen Verabreichung ist die Applikation mittels Medikamentenpumpe bevorzugt. Die orale Applikation und dementsprechend Zubereitungen zur oralen Verabreichung sind besonders bevorzugt.

Vorzugsweise werden erfindungsgemäß bei oraler Applikation je 2 bis 4 Dosiseinheiten täglich, vorzugsweise 2 Dosiseinheiten täglich mit 60 bis 200 mg/Dosiseinheit Morphin, 60 bis 120 mg/ Dosiseinheit Codein, 50 bis 150 mg/Dosiseinheit der Antirheumatika und 1000 bis 2000 mg/Dosiseinheit der Analgetika Metamizol, Acetylsalicylsäure und Paracetamol verabreicht. Stärker bevorzugt werden je 2 Dosiseinheiten täglich mit 80 bis 120 mg/Dosiseinheit Morphin und 80 bis 100 mg/Dosiseinheit Codein, noch stärker bevorzugt 100 mg/Dosiseinheit Morphin und 90 mg/Dosiseinheit Codein verabreicht. De facto muß sich die Dosierung im Einzelfall an der Schmerzintensität des Patienten ausrichten und kann daher von den hier dargestellten Mengen abweichen.

Die Kombination von retardierten und rasch freisetzender Form des jeweiligen Pharmakons kann mittels einer entsprechenden Galenik ermöglicht werden. Hierzu bieten sich folgende Möglichkeiten an:
1. Die Verwendung der bekannten 2-Kammer-Kapseln. Eine Kammer besteht aus einem leicht löslichen, die rasche Freisetzung bewirkenden Material, während die zweite Kammer aus einem langsam freisetzenden Material gebildet wird (vgl. z.B. die DE 35 06 895).
2. Tabletten, die einen Kern mit einer die retardierte Freisetzung bewirkenden Matrix sowie eine Schale enthalten, die mit herkömmlicher Galenik das Pharmakon rasch freisetzt (DE 41 22 039).
3. Eine Mischung aus Pellets (Retardform) und einem Granulat, das entsprechend der üblichen Galenik rasch freisetzt. Die Mischung kann in Weich- bzw. Hartkapseln abgefüllt oder direkt zu Tabletten verpreßt werden.
4. Ein Feingranulat auf Ionenaustauscherbasis (z.B. das von der Firma Bayer unter der Bezeichnung K 1491 vertriebene Produkt) vermischt mit einem rasch freiset zenden Granulat herkömmlicher Galenik, das ebenfalls in Weich- bzw. Hart kapseln abgefüllt oder direkt zu Tabletten verpreßt werden kann.

Die erfindungsgemäßen Zubereitungen können dem Fachmann bekannte Hilfs- und Zusatzstoffe insbesondere, Tablettier- und Granulierhilfen, Binder, Sprengmittel, Aromen, Süßmittel, Harze, Ionenaustauscher etc. enthalten. Sie können weiterhin eine Beschichtung aufweisen, die zur Steuerung der Freisetzung beitragen, d.h. diese verzögern oder beschleunigen kann, oder lediglich zur Maskierung des Geschmacks oder der optischen Kennzeichnung dienen kann.

Bei parenteraler Verabreichung, vorzugsweise mit einer Medikamentenpumpe, liegen die erfindungsgemäß zu verabreichenden Pharmaka in Lösung vor, wobei die Lösung für die parenterale Verabreichung geeignet bspw. isoton ist. Bei der bevorzugten Verabreichung mittels Medikamentenpumpe wird die Abgabe des Pharmakons so eingestellt, daß die oben genannten Wirkverhältnisse und Plasmaspiegel eingehalten werden.

Die erfindungsgemäße pharmazeutische Zubereitung gestattet eine Verminderung der Nebenwirkungen in der Schmerztherapie. Überraschenderweise wurde nämlich gefunden, daß die Schmerzintensitätskurve in 24 Stunden einer circadianen Rhythmik mit ausgesprochenen Spitzenwerten der Schmerzintensität in den frühen Morgenstunden und am frühen Abend unterliegt.

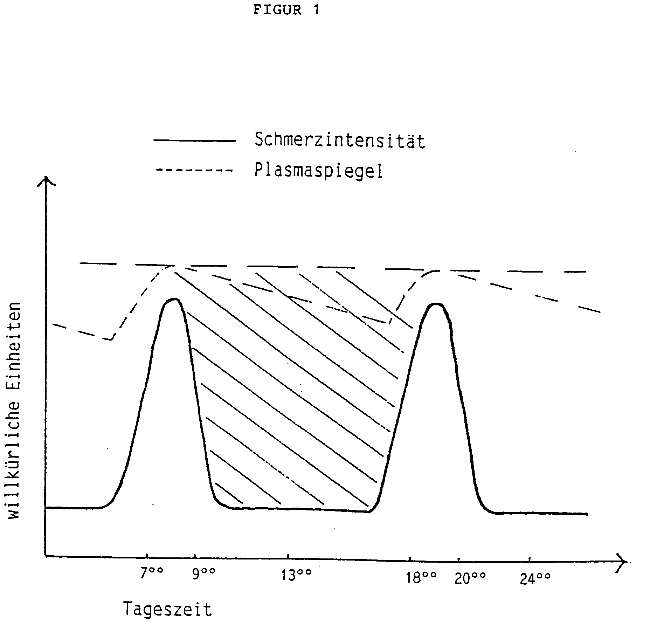

Wie in Fig. 1 dargestellt, deckt die bisherige Schmerztherapie unter Annahme konstanter Schmerzintensitäten (durchgezogene Linie) diese Spitzenwerte der Schmerzintensität mit gleichmäßig hohen Plasmaspiegeln des Pharmakons (gestrichelte Linie) ab. Zu Zeiten geringerer Schmerzintensität z.B. nachts oder in den Mittagsstunden erfolgt dagegen eine deutliche Überdosierung des Pharmakons (schraffierte Fläche in Fig.1). Diese relative Überdosierung im Zeitraum der geringeren Schmerzintensität ist für die beobachteten, unerwünschten Nebenwirkungen des Pharmakons verantwortlich zu machen.

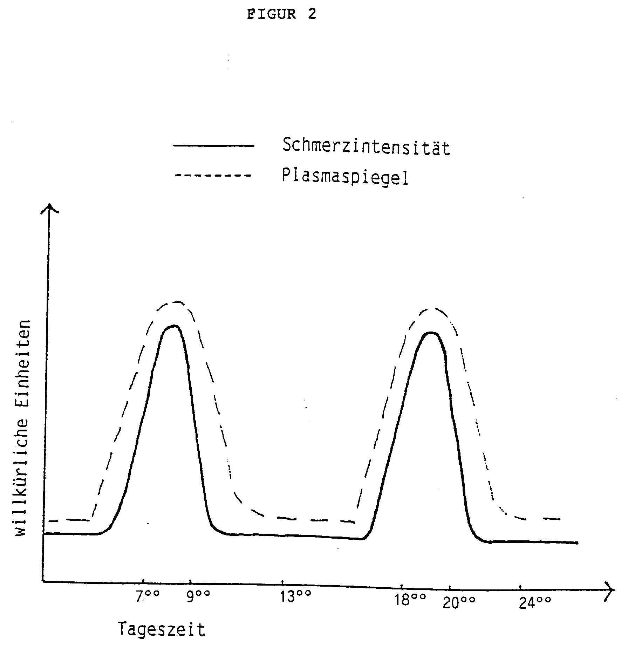

Demgegenüber gestatten es die erfindungsgemäßen pharmazeutischen Zubereitungen, wie in Fig.2 schematisch dargestellt, die Dosierung des Pharmakons der tatsächlichen 24-Stunden-Rhythmik der Schmerzintensität (durchgezogene Linie) anzupassen. So deckt die sofort freisetzende Komponente den Spitzenwert der Schmerzintensität durch entsprechend hohe Plasmakonzentrationen (gestrichelte Linie) des Pharmakons ab. Dagegen wird die geringere Schmerzintensität in den darauf folgenden Stunden durch die retardierte Form des Pharmakons in entsprechend geringerer Dosierung ausreichend abgedeckt.

Durch die zeitlich abgestimmte Freisetzung der erfindungsgemäßen pharmazeutischen Zubereitung wird eine relative Überdosierung des Pharmakons vermieden. Dementsprechend sind auch die Nebenwirkungen des Präparats, insbesondere bei Opioiden und Opiaten verringert.

Entsprechend der circadianen Rhythmik der Schmerzintensität sind erfindungsgemäß die Boluseinheiten auf zweimal tägliche Verabreichung ausgelegt. Die Verabreichung sollte unmittelbar vor Anstieg der Schmerzintensität also in den frühen Morgenstunden und am späten Nachmittag erfolgen.

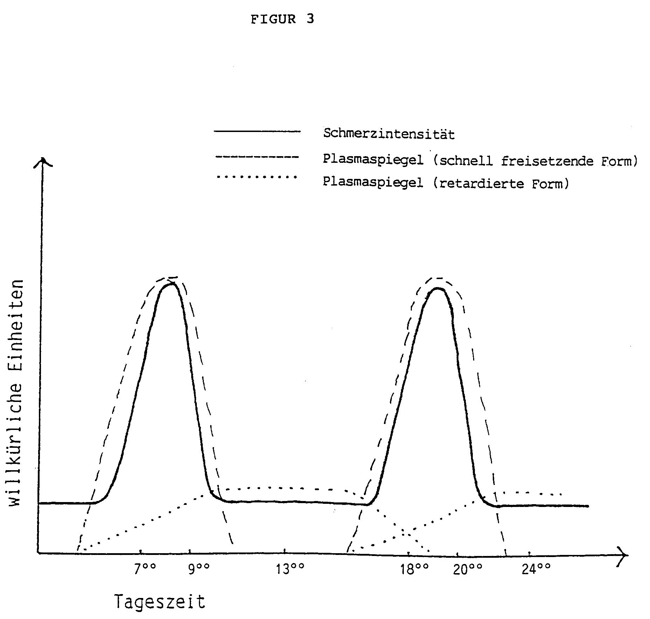

Figur 3 veranschaulicht in schematischer Form die Freisetzung bzw. die daraus resultierenden Plasmaspiegel der schnell freisetzenden und der retardierten Form des Pharmakons. Aus dieser Abbildung ist ersichtlich, daß mit der retardierten Form lediglich die niedrigeren Phasen der Schmerzintensitätskurve abgedeckt werden, während die Spitzenintensitäten durch die schnell freisetzende Form therapiert werden. Durch diese gezielte Verabreichung bzw. Steuerung der Freisetzung lassen sich die Nebenwirkungen der herkömmlichen Schmerztherapie signifikant verringern, da die zu verabreichende Dosis am Pharmakon deutlich gesenkt werden kann bspw. für Morphinderivate um etwa 20 %.

### Beispiel 1: Morphinkapsel

| Retardierte Form: | |
|---|---|
| Feingranulat auf Ionenaustauschbasis (erhältlich von der Firma Bayer unter der Bezeichnung K 1491) | 250 mg |
| Morphin | 80 mg |

| rasch freisetzende Form: | |
|---|---|
| herkömmliches Granulat | 300 mg |
| Morphin | 80 mg |

## Patentansprüche

1. Pharmazeutische Zubereitung enthaltend ein zur Schmerztherapie wirksames Pharmakon in retardierter und rasch freisetzender Form, wobei das Verhältnis von retardierter zu rasch freisetzender Form zwischen 20 : 80 bis 80 : 20 und das Wirkungsverhältnis, bezogen auf die Schmerzintensität, von rasch freisetzender zu retardierender Form bei > 1 : 1 bis 20 : 1 liegt.

2. Pharmazeutische Zubereitung gemäß Anspruch 1, dadurch gekennzeichnet, daß es sich bei dem Pharmakon um ein Analgetikum, ein Antiphlogistikum, ein Antirheumatikum, einen nichtsteroidalen Entzündungshemmer, Opioide und Opiate handelt.

3. Pharmazeutische Zubereitung gemäß Anspruch 1 oder 2, dadurch gekennzeichnet, daß es sich bei dem Pharmakon um ein Morphin oder dessen Derivate, ein Codein oder dessen Derivate, Diclophenac oder dessen Derivate, Metamizol, Tramadol, Tilidin, Methadon, Buprenophin, Acetylsalicylsäure, Ibuprofen oder Paracetamol handelt.

4. Pharmazeutische Zubereitung gemäß Anspruch 1, enthaltend 60 bis 200 mg Morphin pro Dosiseinheit.

5. Pharmazeutische Zubereitung gemäß Anspruch 1, enthaltend 60 bis 120 mg Codein pro Dosiseinheit.

6. Pharmazeutische Zubereitung gemäß Anspruch 4 oder 5, die das Pharmakon in einem Verhältnis von retardierter zu rasch freisetzender Form von 40 : 60 bis 60 : 40, vorzugsweise 50 : 50 enthält.

7. Pharmazeutische Zubereitung gemäß einem der Ansprüche 1 bis 6, die in Form einer 2-Kammer-Kapsel vorliegt.

8. Pharmazeutische Zubereitung gemäß einem der Ansprüche 1 bis 7, die einen Kern mit einer retardiert freisetzenden Matrix und eine Schale enthält, die mit herkömmlicher Galenik das Pharmakon rasch freisetzt.

9. Pharmazeutische Zubereitung gemäß einem der Ansprüche 1 bis 7, die eine Mischung aus retardiert freisetzenden Pellets und einem rasch freisetzendem Granulat üblicher Galenik enthält.

10. Pharmazeutische Zubereitung gemäß einem der Ansprüche 1 bis 7, die ein Feingranulat auf Ionenaustauscherbasis vermischt mit einem rasch freisetzenden Granulat herkömmlicher Galenik enthält.

11. Verfahren zur Schmerztherapie, dadurch gekennzeichnet, daß dem Patienten eine pharmazeutische Zubereitung gemäß einem der Ansprüche 1 bis 10 verabreicht wird.

12. Verfahren gemäß Anspruch 11, bei dem die Zubereitung in Form von zwei Dosiseinheiten in Intervallen von circa 12 Stunden appliziert wird.

13. Verfahren gemäß Anspruch 11, dadurch gekennzeichnet, daß die Verabreichung mit Hilfe einer Medikamentenpumpe erfolgt.
